# FASCICULE DE BREVET EUROPEEN

(11) **EP 2 405 837 B1**
(45) Date de publication et mention de la délivrance du brevet: **23.12.2015**
(21) Numéro de dépôt: 10708247.1
(22) Date de dépôt: 19.02.2010
(51) Int. Cl.: A61B 17/70

(54) **IMPLANT RACHIDIEN À LIAISON ROTULE VERROUILLABLE**
WIRBELSÄULENIMPLANTAT MIT ARRETIERBARER KUGEL-VERBINDUNG
SPINAL IMPLANT WITH A LOCKABLE BALL LINK

(30) Priorité: 12.03.2009 FR 0901138
(43) Date de publication de la demande: 18.01.2012
(73) Titulaire: Euros, 13600 La Ciotat (FR)
(72) Inventeur: GENNARI, Jean-Marie, F-13008 Marseille (FR); CHATAGNIER, Hervé, F-25320 Boussieres (FR); VITAL, Jean-Marc, F-33200 Bordeaux (FR); NOGUES, Laurent, 97410 St Pierre - Réunion (FR); MOUSSEALLARD, hugues, Pascal, F-97410 St Pierre - Réunion (FR); KOUYOUMDJIAN, Pascal, F-30000 Nîmes (FR); TALLET, Jean-Michel, F-13007 Marseille (FR); TISSERAND, Philippe, F-66330 Cabestany (FR)
(74) Mandataire: Orsini, Fabienne
(86) Numéro de dépôt international: PCT/FR2010/000142
(87) Numéro de publication internationale: WO 2010/103198

(56) Documents cités:
- EP-A- 1 839 606
- US-A- 5 443 467
- US-A1- 2007 288 004
- US-B1- 6 254 602

## Description

### DOMAINE TECHNIQUE AUQUEL SE RAPPORTE L'INVENTION

La présente invention concerne de manière générale les implants rachidiens destinés à immobiliser au moins deux vertèbres l'une par rapport à l'autre.

Elle concerne plus particulièrement un implant rachidien tel que défini dans le préambule de la revendication 1.

### ARRIERE-PLAN TECHNOLOGIQUE

Les implants rachidiens du type précité sont utilisés par paire et en combinaison avec une tige de liaison pour traiter des arthroses, des fractures vertébrales ou pour corriger des déviations de la colonne vertébrale telles que les scolioses ou les cyphoses.

Afin d'immobiliser deux vertèbres, un chirurgien utilise quatre implants rachidiens, à raison de deux par vertèbre. Il engage à cet effet leurs parties d'ancrage dans les vertèbres, puis il solidarise les implants par paire au moyen de deux tiges de liaison qui s'étendent chacun entre deux implants, d'une vertèbre à l'autre.

Ces deux tiges de liaison sont préalablement cintrées en fonction de la correction à apporter à la colonne vertébrale. De cette manière, les deux tiges se positionnent parallèlement à la colonne vertébrale pour la maintenir sensiblement droite.

On connaît plus particulièrement deux types d'implants rachidiens.

On connaît des implants mono-axiaux dans lesquels les parties d'ancrage et de montage sont alignées. Dans la majorité de ces implants, les parties d'ancrage et de montage sont fixes l'une par rapport à l'autre et sont formées d'une seule pièce. Certains de ces implants mono-axiaux sont toutefois agencés de telle sorte que leurs parties d'ancrage et de montage sont liées l'une à l'autre par une liaison pivot autour de l'axe longitudinal de l'implant.

On connaît également des implants poly-axiaux dans lesquels les parties d'ancrage et de montage sont liées l'une à l'autre par une liaison rotule.

On entend ici par « liaison rotule » une liaison qui autorise la partie de montage à prendre différentes inclinaisons par rapport à l'axe de la partie d'ancrage. Les parties d'ancrage et de montage sont ainsi fixes en translation l'une par rapport à l'autre suivant les trois axes de l'espace, mais libres en rotation autour de chacun de ces trois axes.

On entend par ailleurs par « liaison pivot » une liaison qui autorise la partie de montage à tourner autour de la partie d'ancrage en restant alignée selon cet axe. Les parties d'ancrage et de montage sont ainsi fixes en translation l'une par rapport à l'autre suivant les trois axes de l'espace, fixes en rotation l'une par rapport à l'autre autour de deux de ces trois axes, et libres en rotation autour de l'axe de la partie d'ancrage.

Le chirurgien peut être amené à utiliser l'un ou l'autre de ces deux types d'implants rachidiens, en fonction par exemple du traumatisme à soigner et de la forme de la colonne vertébrale du patient.

L'inconvénient est alors que le fabricant d'implants doit doubler ses références pour offrir au chirurgien les deux types d'implants rachidiens qui lui sont nécessaires pour opérer les patients.

Le chirurgien doit quant à lui gérer les stocks de deux types d'implants et prévoir, avant chaque opération, quel type d'implants rachidiens il utilisera.

Un exemple d'implant polyaxial est présenté dans le document EP 1 869 606. Tel que présenté dans ce document, l'implant comporte :
- une vis dont la tête est en partie sphérique,
- une partie de réception tubulaire comportant, du côté de son extrémité basse, une ouverture d'accueil de la tête de la vis,
- un élément de pression tubulaire engagé à l'intérieur de la partie de réception de telle sorte que son extrémité basse appuie contre la tête de la vis, dont l'extrémité haute forme un berceau d'accueil d'une tige de liaison, et
- un dispositif de fermeture comprenant, d'une part, un boulon extérieur vissé dans l'extrémité haute de la partie de réception tubulaire afin de bloquer l'élément de pression contre la tête de la vis de manière à l'immobiliser, et, d'autre part, un boulon intérieur vissé dans le boulon extérieur pour bloquer la tige de liaison au fond du berceau prévu dans l'élément de pression.

Dans ce document, les moyens de blocage de la liaison rotule prévue entre la tête de la vis et l'ouverture d'accueil de la partie de réception tubulaire sont donc situés au-dessus du berceau d'accueil de la tige de liaison.

L'inconvénient de cet implant est alors que la liaison rotule ne peut être bloquée qu'après que la tige de liaison a été engagée dans l'implant. Le chirurgien ne peut donc pas la bloquer avant d'opérer le patient.

On connaît par ailleurs du document US 2007/0288004 un implant rachidien comprenant :
- une vis comportant une tête partiellement sphérique,
- une tulipe tubulaire, définissant intérieurement un logement axial qui est destiné à accueillir transversalement une tige de liaison et qui présente une partie haute taraudée et une partie basse sphérique,
- une rosette qui est adaptée à se loger dans la partie basse sphérique de la tulipe,
- un verrou à visser dans le taraudage de la partie haute de la tulipe pour bloquer la tige de liaison contre la rosette, ce qui a pour effet de comprimer cette dernière dans la partie basse sphérique de la tulipe et d'immobiliser ainsi la vis par rapport à la tulipe.

La rosette présente un téton adapté à s'engager dans l'empreinte de la tête de la vis, de manière qu'une fois la tulipe assemblée à la vis, l'implant se comporte comme un implant monoaxial. Cette rosette peut par ailleurs être remplacée par une autre rosette dépourvue de téton, de manière que l'implant puisse se comporter comme un implant polyaxial.

Toutefois, dans ce document, les moyens permettant à la tête de pivoter par rapport à la tulipe et de bloquer la polyaxialité sont portés par une seule et unique pièce, la rosette. De ce fait, pour passer d'un implant monoaxial à un implant polyaxial, il est nécessaire de retirer l'ensemble de la rosette, et donc de démonter l'implant. Le chirurgien ne peut donc pas bloquer la polyaxialité pendant l'opération du patient.

Le document US 5,443,467 A divulgue un implant tel que défini dans le préambule de la revendication 1.

### OBJET DE L'INVENTION

Le but de la présente invention est de proposer un implant rachidien pouvant aussi bien assurer la fonction d'un implant mono-axial que celle d'un implant poly-axial, afin de faciliter le travail du chirurgien et d'éviter une duplication des frais de fabrication, de stockage et de gestion de ces implants.

A cet effet, on propose selon l'invention un implant rachidien tel que défini dans la revendication 1.

Ainsi, grâce à l'invention, en l'absence d'élément de blocage en position de blocage, l'implant se comporte comme un implant poly-axial. En revanche, lorsqu'un élément de blocage est positionné en position de blocage, l'implant se comporte comme un implant mono-axial. Par conséquent, un seul type d'implants rachidiens permet au chirurgien de soigner l'ensemble des traumatismes de la colonne vertébrale.

Grâce à l'invention, le fabricant et le chirurgien n'ont à gérer qu'une seule référence d'implants rachidiens.

Par ailleurs, le chirurgien peut choisir quel type d'implant il souhaite mettre en place sur la colonne vertébrale du patient au cours de l'opération chirurgicale elle-même, au bénéfice de la facilité d'utilisation de cet implant.

En particulier, il peut configurer son implant en mode mono-axial soit avant l'opération du patient, soit durant l'opération. Il peut même le configurer ainsi après avoir engagé la partie d'ancrage de l'implant dans la colonne vertébrale du patient. Il peut également choisir de conserver la poly-axialité de son implant, de manière à immobiliser la liaison rotule seulement à la fin de l'opération, lorsqu'il engage la tige de liaison dans le logement axial de l'implant.

D'autres caractéristiques avantageuses et non limitatives de l'implant rachidien selon l'invention définies dans les revendications 2 et suivantes

### DESCRIPTION DÉTAILLÉE D'UN EXEMPLE DE RÉALISATION

La description qui va suivre, en regard des dessins annexés, donnée à titre d'exemple non limitatif, fera bien comprendre en quoi consiste l'invention et comment elle peut être réalisée.

Sur les dessins annexés :
- la figure 1 est une vue en perspective de deux vertèbres bloquées l'une par rapport à l'autre à l'aide d'une tige de liaison et de deux implants rachidiens selon l'invention ;
- la figure 2 est une vue éclatée d'un premier mode de réalisation de l'un des implants rachidiens de la figure 1 ;
- la figure 3 est une vue en coupe axiale de l'implant rachidien de la figure 2, dans lequel la liaison rotule est laissée libre ;
- les figures 4 et 5 sont des vues en perspective de l'implant rachidien de la figure 3, seul ou équipé d'une vis de verrouillage de la tige de liaison ;
- les figures 6 à 8 sont des schémas de fonctionnement du blocage de l'implant rachidien de la figure 2 ;
- la figure 9 est une vue en coupe axiale de l'implant rachidien de la figure 2, dans lequel la liaison rotule est bloquée en liaison pivot ;
- les figures 10 et 11 sont des vues en perspective de l'implant rachidien de la figure 9, seul ou équipé d'une vis de verrouillage de la tige de liaison ;
- la figure 12 est une vue éclatée d'un second mode de réalisation de l'un des implants rachidiens de la figure 1 ;
- la figure 13 est une vue en perspective de l'implant rachidien de la figure 12 et d'une clef de blocage ;
- la figure 14 est une vue en coupe axiale de l'implant rachidien de la figure 12, dans lequel la liaison rotule est laissée libre ;
- la figure 15 est une vue en coupe axiale de l'implant rachidien de la figure 14 et de la clef de blocage, dans lequel la liaison rotule est bloquée en liaison pivot ;
- la figure 16 est une vue éclatée d'un troisième mode de réalisation de l'un des implants rachidiens de la figure 1 ;
- la figure 17 est une vue en coupe axiale de l'implant rachidien de la figure 16, dans lequel la liaison rotule est laissée libre ;
- la figure 18 est une vue en coupe axiale de l'implant rachidien de la figure 16, dans lequel la liaison rotule est bloquée en liaison pivot ;
- la figure 19 est une vue éclatée d'un quatrième mode de réalisation de l'un des implants rachidiens de la figure 1 ;

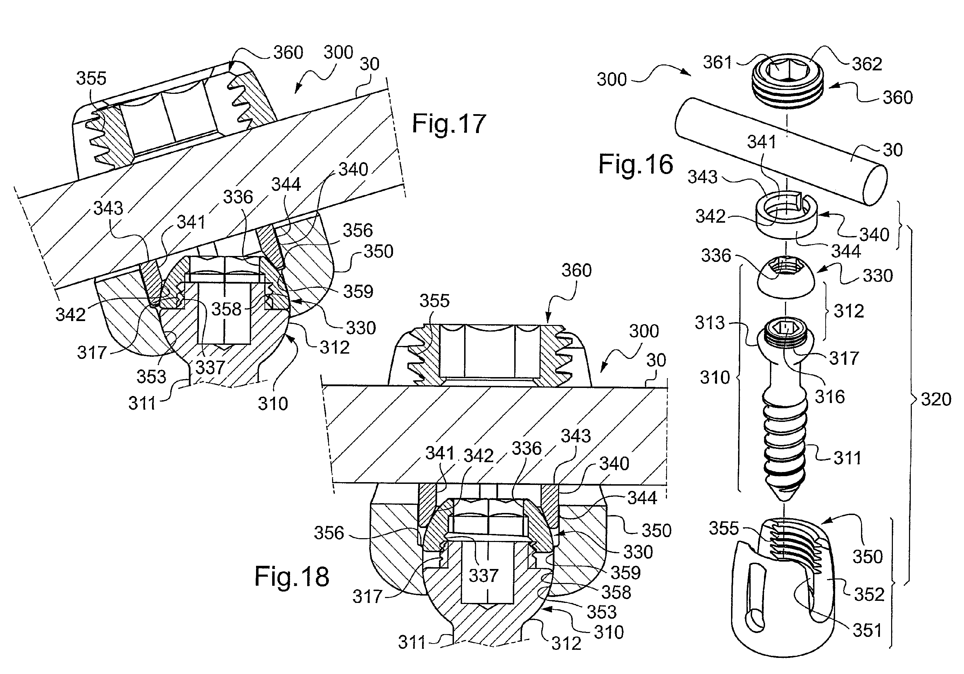

- la figure 17 est une vue en coupe axiale de l'implant rachidien de la figure 16, dans lequel la liaison rotule est laissée libre ;
- la figure 18 est une vue en coupe axiale de l'implant rachidien de la figure 16, dans lequel la liaison rotule est bloquée en liaison pivot ;

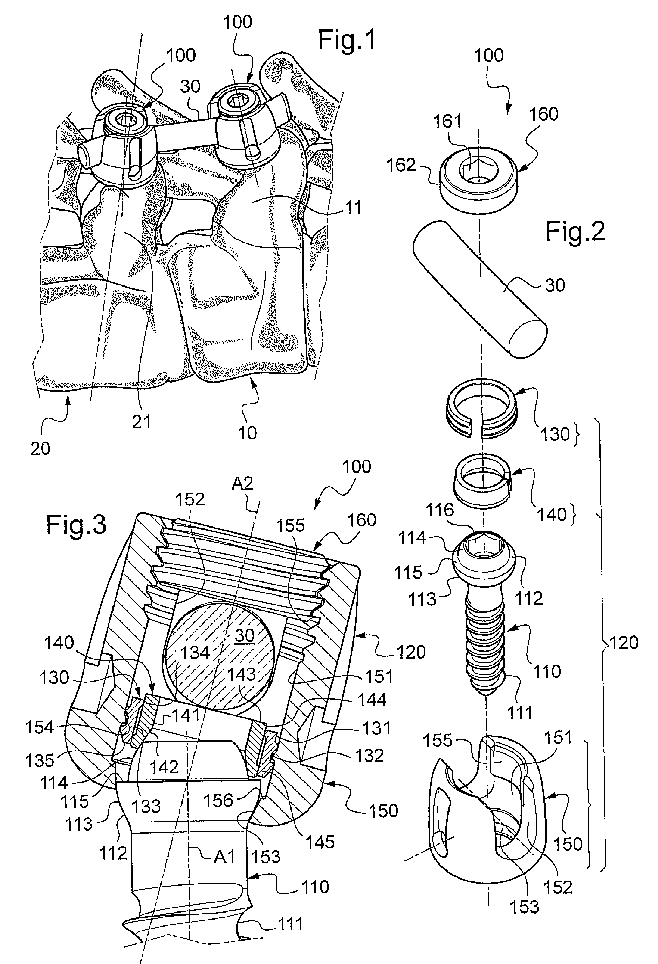

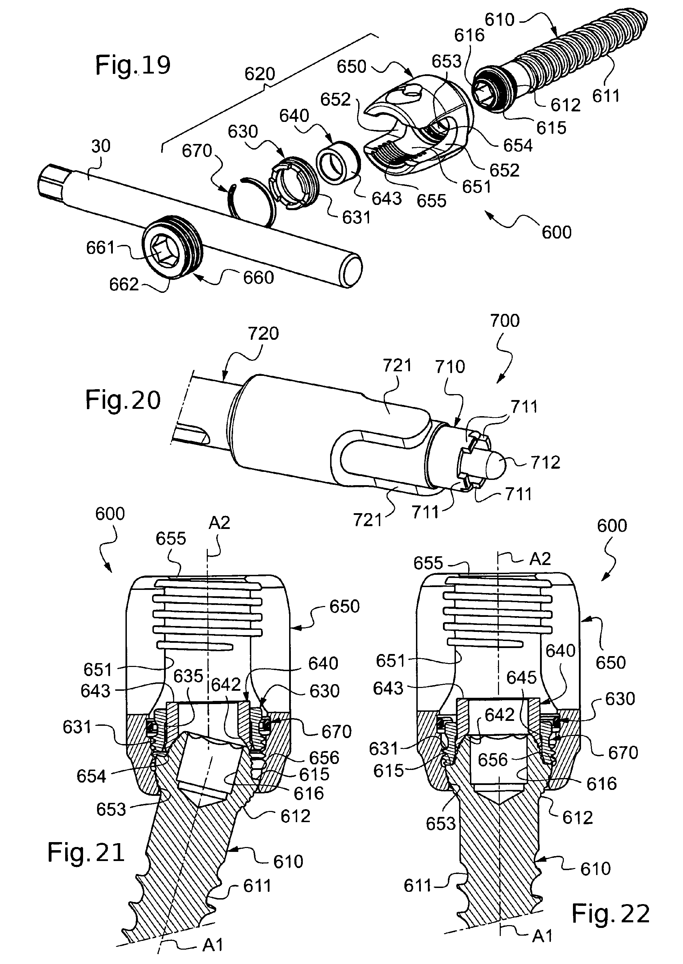

- la figure 19 est une vue schématique éclatée d'un quatrième mode de réalisation de l'un des implants rachidiens de la figure 1 ;
- la figure 20 est une vue schématique en perspective d'un outil de blocage de l'implant rachidien de la figure 19 ;
- la figure 21 est une vue en coupe axiale de l'implant rachidien de la figure 19, dans lequel la liaison rotule est laissée libre ; et
- la figure 22 est une vue en coupe axiale de l'implant rachidien de la figure 19, dans lequel la liaison rotule est bloquée.

On a respectivement représenté sur les figures 2 à 11, 12 à 15, 16 à 18, et 19 à 22 quatre modes de réalisation d'un implant rachidien 100 ; 200 ; 300 ; 600.

Tel que représenté sur ces figures, l'implant rachidien 100 ; 200 ; 300 ; 600 est une vis pédiculaire qui comporte une partie d'ancrage 110 ; 210 ; 310 ; 610 à ancrer par vissage dans le pédicule 21, 11 d'une vertèbre 10, 20, et une partie de montage 120 ; 220 ; 320 ; 620 apte à accueillir une tige de liaison 30.

Comme le montre la figure 1, une telle tige de liaison 30 permet, lorsqu'elle s'étend entre deux implants rachidiens 100 ancrés à deux vertèbres 10, 20, d'immobiliser ces deux vertèbres 10, 20 l'une par rapport à l'autre.

Dans les quatre modes de réalisation de l'implant rachidien 100 ; 200 ; 300 ; 600 (figures 2, 12, 16, et 19), la partie d'ancrage 110 ; 210 ; 310 ; 610 présente une forme de vis, avec un corps fileté 111 ; 211 ; 311 ; 611 d'axe A1 et, à l'extrémité arrière de ce corps fileté, une tête 112 ; 212 ; 312 ; 612 présentant une empreinte de réception 116 ; 216 ; 316 ; 616 de l'extrémité d'un outil de vissage. Ici, cette empreinte de réception 116 ; 216 ; 316 ; 616 est une empreinte hexagonale apte à coopérer avec une clef allen.

Dans ces quatre modes de réalisation, la partie de montage 120 ; 220 ; 320 ; 620 de l'implant rachidien 100 ; 200 ; 300 ; 600 comporte un corps 150 ; 250 ; 350 ; 650 allongé, globalement cylindrique autour d'un axe A2.

Ce corps 150 ; 250 ; 350 ; 650 délimite intérieurement un logement axial 151 ; 251 ; 351 ; 651 cylindrique de révolution autour de l'axe A2. Du côté de la partie d'ancrage 110; 210; 310; 610, ce logement axial est délimité par un fond 156 ; 256 ; 356 ; 656. Ce logement axial 151 ; 251 ; 351 ; 651 débouche à l'extérieur, à l'arrière de l'implant rachidien 100 ; 200 ; 300 ; 600, par une ouverture d'introduction 155 ; 255 ; 355 ; 655. Il débouche par ailleurs vers l'avant par une ouverture 153 ; 253 ; 353 ; 653 prévue dans le fond 156; 256; 356; 656 du corps, dans laquelle est engagée la tête 112 ; 212 ; 312 ; 612 de la partie d'ancrage 110 ; 210 ; 310 ; 610.

Le corps 150; 250; 350; 650 est par ailleurs muni de deux ouvertures latérales 152 ; 252 ; 352 ; 652 en regard l'une de l'autre qui s'étendent en longueur parallèlement à l'axe A2 et qui débouchent à l'arrière de l'implant rachidien 100; 200; 300; 600. Les fonds de ces ouvertures latérales 152 ; 252 ; 352 ; 652, qui s'étendent vers l'avant de l'implant rachidien 100 ; 200 ; 300 ; 600, sont arrondis et présentent ainsi des formes de berceaux. Ces deux ouvertures latérales 152 ; 252 ; 352 ; 452 ; 652 permettent d'engager la tige de liaison 30 transversalement dans le logement axial 151 ; 251 ; 351 ; 651 du corps 150 ; 250 ; 350 ; 650.

L'implant rachidien 100 ; 200 ; 300 ; 600 comporte par ailleurs des moyens de verrouillage 160 ; 260 ; 360 ; 660 destinés à coopérer avec le corps 150 ; 250 ; 350 ; 650 pour bloquer la tige de liaison 30 dans le corps 150 ; 250 ; 350 ; 650.

Ces moyens de verrouillage comprennent une vis de verrouillage 160 ; 260 ; 360 ; 660 présentant un corps fileté 162 ; 262 ; 362 ; 662 muni, en creux dans sa face arrière accessible au chirurgien, d'une empreinte de réception 161 ; 261 ; 361 ; 661 de l'extrémité d'un outil de vissage. Ici, cette empreinte de réception 161 ; 261 ; 361 ; 661 est hexagonale et est apte à coopérer avec une clef allen.

Le corps fileté 162 ; 262 ; 362 ; 662 de cette vis de verrouillage 160 ; 260 ; 360 ; 660 est apte à être vissé dans un taraudage prévu au niveau de l'ouverture d'introduction 155 ; 255 ; 355 ; 655 du logement axial 151 ; 251 ; 351 ; 651 du corps 150 ; 250 ; 350 ; 650 de la partie de montage 120 ; 220 ; 320 ; 620.

Lorsque la vis de verrouillage 160 ; 260 ; 360 ; 660 est vissée dans ce taraudage, elle s'enfonce dans le logement axial 151 ; 251 ; 351 ; 651 pour prendre appui contre la tige de liaison 30 afin de la bloquer dans le corps 150 ; 250 ; 350 ; 650 de la partie de montage 120 ; 220 ; 320 ; 620.

Selon les modes de réalisation représentés sur les différentes figures, comme cela sera décrit plus en détail dans la suite de cet exposé, la partie d'ancrage 110 ; 210 ; 310 ; 610 et la partie de montage 120 ; 220 ; 320 ; 620 de l'implant rachidien 100 ; 200 ; 300 ; 600 comprennent respectivement des premiers et des seconds moyens de liaison qui coopèrent ensemble de telle manière que les parties d'ancrage et de montage sont liées ensemble par une liaison rotule.

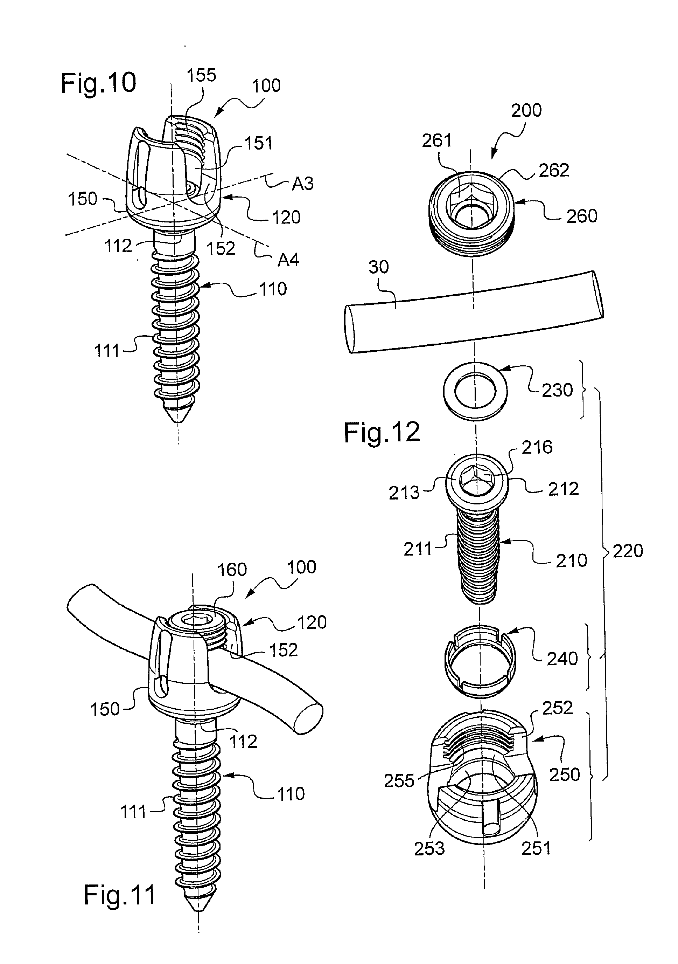

Selon une caractéristique particulièrement avantageuse de l'invention, l'implant rachidien 100 ; 200 ; 300 ; 600 comprend par ailleurs des moyens de retenue 154 ; 245 ; 317 ; 654 situés à proximité du fond 156 ; 256 ; 356 ; 456 ; 656 du corps 150 ; 250 ; 350 ; 650, qui sont adaptés à fixer un élément de blocage 130 ; 230 ; 330 ; 630 dans une position fixe dite de blocage, dans laquelle la liaison rotule formée par lesdits premiers et seconds moyens de liaison est bloquée pour rendre lesdites parties d'ancrage 110 ; 210 ; 310 ; 610 et de montage 120 ; 220 ; 320 ; 620 fixes en rotation l'une par rapport à l'autre autour d'au moins deux axes orthogonaux A3, A4 (voir figure 10).

Les moyens de retenue 154 ; 245 ; 317 ; 654 sont situés dans un espace situé entre le fond 156 ; 256 ; 356 ; 656 du corps 150 ; 250 ; 350 ; 650 et le fond des ouvertures latérales 152 ; 252 ; 352 ; 652 en forme de berceau, cet espace incluant le fond 156 ; 256 ; 356 ; 656 du corps 150 ; 250 ; 350 ; 650 lui même et une partie du logement axial 151 ; 251 ; 351 ; 651.

Les moyens de retenue 154 ; 245 ; 317 ; 654 sont quoi qu'il en soit situés de telle sorte que le chirurgien peut y avoir accès et intervenir sur eux indépendamment de la tige de liaison 30. Le chirurgien peut ainsi bloquer la liaison rotule lorsqu'il le souhaite, avant d'opérer le patient ou au cours de l'opération, avant de rapporter la tige de liaison 30 dans le logement axial 151 ; 251 ; 351 ; 651.

Ici, les moyens de retenue 154; 245; 317; 654 sont plus précisément situés entre le fond 156 ; 256 ; 356 ; 656 du corps 150 ; 250 ; 350 ; 650 et les fonds des ouvertures latérales 152 ; 252 ; 352 ; 652 du corps 150 ; 250 ; 350 ; 650.

Dans les trois premiers modes de réalisation de l'implant rachidien 100 ; 200 ; 300 (figures 1 à 18), le blocage de la liaison rotule permet de transformer cette liaison rotule en liaison pivot. De cette manière, lorsque la liaison rotule est bloquée, les parties d'ancrage 110 ; 210 ; 310 et de montage 120 ; 220 ; 320 sont libres de pivoter autour d'un seul et même axe, appelé axe principal A1.

Dans ces trois modes de réalisation, les moyens de retenue 154 ; 245 ; 317 sont plus précisément agencés de telle manière qu'ils sont capables de fixer ledit élément de blocage 130 en position de blocage seulement lorsque l'axe A2 du corps 150 ; 250 ; 350 est confondu avec l'axe A1 de la partie d'ancrage 110 ; 210 ; 310, qui constitue ainsi ledit axe principal.

Dans les quatrième modes de réalisation de l'implant rachidien 600 (figures 19 à 22), le blocage de la liaison rotule permet d'immobiliser les parties d'ancrage 610 et de montage 620 l'une par rapport à l'autre. Dans ce quatrième mode de réalisation, les moyens de retenue 654 sont agencés de telle manière qu'ils sont capables de fixer ledit élément de blocage 630 en position de blocage seulement lorsque l'axe A2 du corps 650 est confondu avec l'axe A1 de la partie d'ancrage 610.

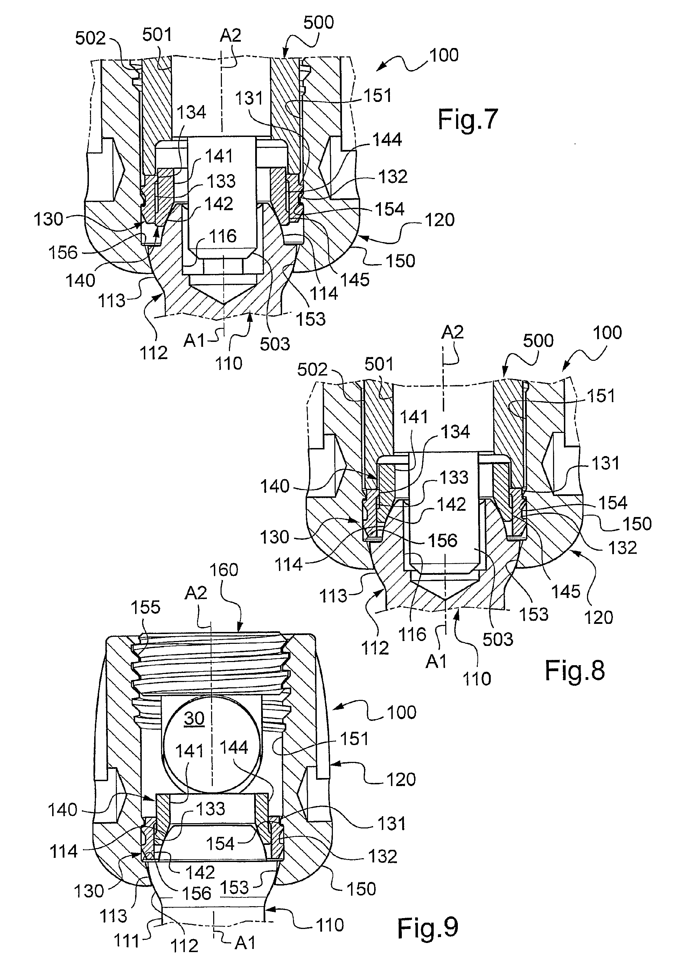

Ainsi, dans chacun de ces quatre modes de réalisation, lorsqu'aucun élément de blocage n'est retenu en position de blocage par les moyens de retenue 154 ; 245 ; 317 ; 654, l'implant 100 ; 200 ; 300 ; 600 se comporte comme un implant poly-axial puisque la liaison rotule est laissée libre (on dit alors de la liaison rotule qu'elle est activée - figures 3, 14, 17, et 21). En revanche, lorsque les moyens de retenue 154 ; 245 ; 317 ; 654 fixent un élément de blocage 130 ; 230 ; 330 ; 630 en position de blocage, ce même implant 100; 200; 300; 600 se comporte comme un implant mono-axial puisque la liaison rotule est soit complètement bloquée, soit bloquée en liaison pivot (on dit alors de la liaison rotule qu'elle est désactivée - figures 9, 15, 18, et 22).

Avantageusement, les moyens de retenue 154 ; 245 ; 317 ; 654 sont en outre adaptés à stocker l'élément de blocage 130 ; 230 ; 330 ; 630 dans une position fixe dite de stockage, distincte de la position de blocage, dans laquelle la liaison rotule est laissée active.

Grâce à ces moyens de retenue 154 ; 245 ; 317 ; 654, le fabricant d'implants peut livrer l'implant rachidien 100 ; 200 ; 300 ; 600 avec l'élément de blocage 130 ; 230 ; 330 ; 630 bloqué en position de stockage, de manière que celui-ci soit non seulement imperdable mais également facilement accessible au chirurgien si ce dernier souhaite désactiver la liaison rotule.

Préférentiellement, les premiers moyens de liaison sont formés par la tête 112; 212 ; 312 ; 612 de la partie d'ancrage 110 ; 210 ; 310 ; 610 qui est au moins en partie sphérique pour former l'embout mâle de la liaison rotule.

Les seconds moyens de liaison comportent quant à eux une bague 140 ; 240 ; 340 ; 640 qui est logée dans le logement axial 151 ; 251 ; 351 ; 651 du corps 150 ; 250 ; 350 ; 650 de la partie de montage 120 ; 220 ; 320 ; 620.

Cette bague 140 ; 240 ; 340 ; 640 présente une face intérieure 142 ; 242 ; 342 ; 642 en appui contre la tête 112 ; 212 ; 312 ; 612 de la partie d'ancrage 110 ; 210 ; 310 ; 610 pour former une partie au moins de l'embout femelle de la liaison rotule.

La bague 140 ; 240 ; 340 est préférentiellement fendue longitudinalement sur au moins une partie de sa longueur par au moins une fente, ce qui lui confère une élasticité radiale pour qu'elle puisse s'adapter à la forme de la tête 112 ; 212 ; 312 de la partie d'ancrage 110 ; 210 ; 310.

La bague 140 ; 240 ; 340 ; 640 présente une face d'extrémité 143 ; 243 ; 343 ; 643 qui s'étend à l'arrière des fonds des berceaux formés par les deux ouvertures latérales 152 ; 252 ; 352 ; 652 du corps 150 ; 250 ; 350 ; 650 de la partie de montage 120 ; 220 ; 320 ; 620. Autrement formulé, la bague 140 ; 240 ; 340 ; 640 s'étend vers l'ouverture d'introduction 155 ; 255 ; 355 ; 655 du logement axial 151 ; 251 ; 351 ; 651 du corps 150 ; 250 ; 350 ; 650, de telle manière qu'une très faible partie de sa longueur est située entre cette ouverture d'introduction 155 ; 255 ; 355 ; 655 et les fonds des deux ouvertures latérales 152 ; 252 ; 352 ; 652 du corps 150 ; 250 ; 350 ; 650.

Ainsi, lorsque le chirurgien serre la vis de verrouillage 160 ; 260 ; 360 ; 660 dans le taraudage prévu au niveau de l'ouverture d'introduction 155 ; 255 ; 355 ; 655 du logement axial 151 ; 251 ; 351 ; 651, cette vis de verrouillage vient appuyer sur la tige de liaison 30 qui appuie elle-même sur la face arrière 143 ; 243 ; 343 ; 643 de la bague 140 ; 240 ; 340 ; 640, ce qui bloque tout mouvement relatif de la bague par rapport à la tête 112 ; 212 ; 312 ; 612 de la partie d'ancrage 110 ; 210 ; 310 ; 610. Ainsi, cette vis de verrouillage 160; 260; 360 ; 660 permet d'immobiliser la partie de montage 120 ; 220 ; 320 ; 620 par rapport à la partie d'ancrage 110 ; 210 ; 310 ; 610, de telle manière que l'axe A2 présente l'orientation voulue par rapport à l'axe A1.

Dans les premier, second, et quatrième modes de réalisation de l'implant rachidien 100 ; 200 ; 600, respectivement représentés sur les figures 2 à 11, 12 à 15, et 19 à 22, l'élément de blocage 130 ; 230 ; 630 appartient à la partie de montage 120 ; 220 ; 620.

Il est plus précisément constitué par un anneau logé dans le logement axial 151 ; 251 ; 651 du corps 150 ; 250 ; 650 de la partie de montage 120 ; 220 ; 620 et réalisé, à l'instar de l'ensemble des autres éléments de l'implant, en titane.

Dans les premier, second et quatrième modes de réalisation, l'élément de blocage 130; 230; 630 et la partie d'ancrage 110; 210 ; 610 comportent des faces de butée qui, lorsque ledit élément de blocage 130 ; 230 ; 630 est en position de blocage, sont situées au contact ou à proximité immédiate l'une de l'autre, ce qui désactive la liaison rotule.

Plus particulièrement, dans le premier mode de réalisation de l'invention représenté sur les figures 2 à 11, la tête 112 de la partie d'ancrage 110 présente deux parties hémisphériques 113, 114, dont une partie hémisphérique avant 113 dont le sommet se raccorde au corps fileté 111 de la partie d'ancrage 110, et une partie hémisphérique arrière 114 dont le sommet est tourné vers la partie de montage 120. Cette partie hémisphérique arrière 114 présente un diamètre inférieur à celui de la partie hémisphérique avant 113. Cette différence de diamètres entre les deux parties hémisphériques 113, 114 génère sur la tête 112 de la partie d'ancrage 110 un épaulement annulaire 115 autour de l'axe A1.

En correspondance, l'ouverture 153 située au fond du logement axial 151 du corps 150 de la partie de montage 120 présente une forme soit tronconique, soit de tronçon de sphère (ou « tranche de sphère ») de diamètre égal, au jeu près, au diamètre de la partie hémisphérique avant 113 de la tête 112 de la partie d'ancrage 110. Cette ouverture 153 forme ainsi une partie desdits seconds moyens de liaison. Elle forme en d'autres termes une partie de l'embout femelle de la liaison rotule.

Telle que représentée sur la figure 3, la bague 140 présente ici une forme sensiblement tubulaire, avec une face extérieure 144 cylindrique de révolution autour de l'axe A2 qui est bordée, du côté de son extrémité avant, par une couronne extérieure 145 de plus grand diamètre. La bague 140 présente par ailleurs une face intérieure en deux parties, dont une partie arrière 141 cylindrique de révolution autour de cet axe A2, et une partie avant 142 tronconique engagée sur la partie hémisphérique arrière 114 de la tête 112 de la partie d'ancrage 110.

L'élément de blocage 130 présente une forme d'anneau et est engagé sur la face extérieure 144 de la bague 140. Il est à cet effet fendu longitudinalement, ce qui lui confère une élasticité radiale pour s'adapter au diamètre extérieur de la bague 140. L'élément de blocage 130 présente une face intérieure 133 cylindrique de révolution autour de l'axe A2. Il présente un diamètre intérieur égal, au jeu près, au diamètre extérieur de la couronne 145 de la bague 140. Il est bordé, à son extrémité arrière, par une couronne intérieure 134. Il est ainsi adapté à coulisser le long de la face extérieure 144 de la bague 140 jusqu'à une position de butée dans laquelle sa couronne intérieure 134 vient au contact de la couronne extérieure 145 de la bague 140.

Dans ce mode de réalisation de l'invention, les moyens de retenue 154 de l'élément de blocage 130 viennent de formation avec le corps 150 de la partie de montage 120. Ils sont en l'espèce formés par une nervure périphérique 154 qui s'étend dans le logement axial 151, en saillie de la face intérieure de ce logement axial 151, et qui présente ici une symétrie de révolution autour de l'axe A2.

L'élément de blocage 130 présente en correspondance, sur sa face extérieure, au moins une rainure périphérique 131, 132 à engager sur ladite nervure périphérique 154 pour être fixé en position de blocage.

Tel que représenté sur les figures, l'élément de blocage 130 présente deux dents d'encliquetage, qui définissent deux rainures périphériques 131, 132 de révolution autour de l'axe A2. Ces deux rainures périphériques 131, 132 permettant de bloquer l'élément de blocage 130 sur la nervure périphérique 154 du corps 150, au choix, en position de stockage (figure 3) ou de blocage (figure 9).

Dans la position de stockage, la face avant 135 de l'élément de blocage 130 est située à distance de l'épaulement annulaire 115 de la tête 112 de la partie d'ancrage 110, de manière à laisser la liaison rotule activée.

En revanche, en position de blocage, la face avant 135 de l'élément de blocage 130 est située au contact ou à proximité immédiate de l'épaulement annulaire 115 de la tête 112 de la partie d'ancrage 110, de manière à désactiver la liaison rotule. La face avant 135 de l'élément de blocage 130 et l'épaulement annulaire 115 de la tête 112 de la partie d'ancrage 110 forment ainsi des faces de butée, chacune symétrique de révolution autour des axes A1 et A2 confondus.

Par « proximité immédiate », on entend que ces deux faces de butée sont situées à une distance telle l'une de l'autre qu'elles empêchent tout débattement de la partie de montage 120 par rapport à la partie d'ancrage 110 de plus de 5 degrés autour d'un axe orthogonal à l'axe principal A1.

Ici, la rainure périphérique 154 présente une section en forme de triangle rectangle dont l'hypoténuse est tournée vers l'arrière du corps 150 de la partie de montage 120, de manière que l'élément de blocage 130 peut être engagé par l'arrière du corps 150 à l'intérieur du logement axial 151, en position de stockage puis de blocage, mais qu'il ne peut ni passer de la position de blocage à la position de stockage, ni être extrait du corps 150. Il est ainsi parfaitement imperdable.

La mise en place de l'implant rachidien 100 sur une vertèbre 10, 20 est réalisée par le chirurgien de la manière suivante.

Lorsque le chirurgien s'empare d'un nouvel implant rachidien 100, celui-ci est préférentiellement déjà équipé, d'une part, d'un élément de blocage 130 disposé en position de stockage, et, d'autre part, d'une vis de verrouillage 160 vissée dans le corps 150 de la partie de montage 120.

Le chirurgien commence alors par extraire cette vis de verrouillage 160 du corps 150. Ayant accès à l'empreinte de réception 116 de la tête 112 de la partie d'ancrage 110 de l'implant 100, il visse cette partie d'ancrage 110 dans la vertèbre 10, 20 du patient.

Il estime ensuite s'il faut ou non désactiver la liaison rotule de l'implant rachidien 100 pour maintenir au mieux la colonne vertébrale du patient.

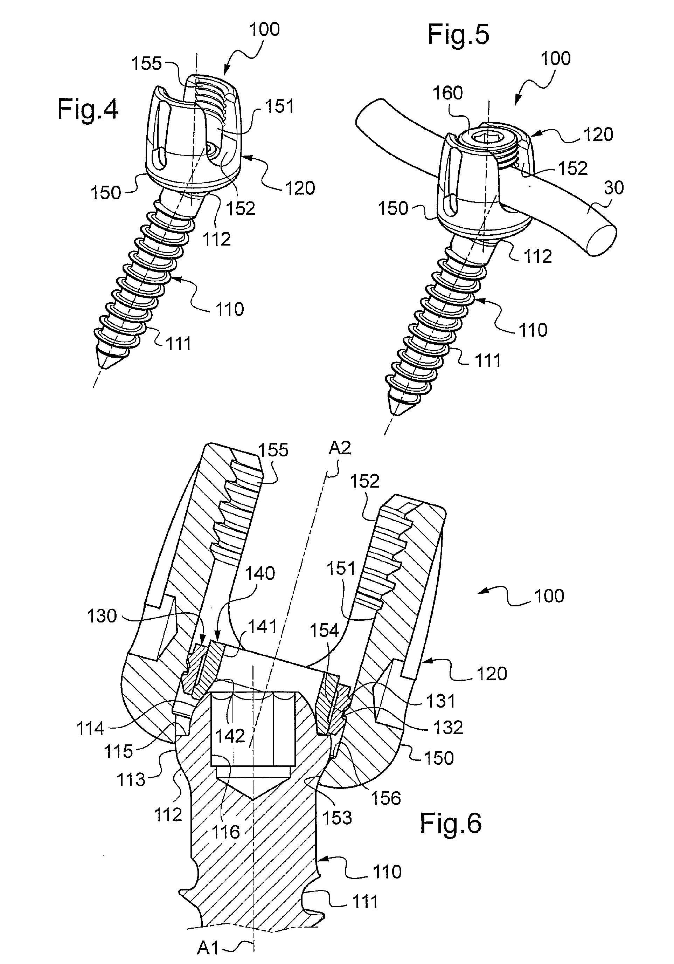

S'il souhaite laisser la liaison rotule active (figures 4 et 6), il place la tige de liaison 30 préalablement cintrée dans le logement axial 151 du corps 150 de la partie de montage 120, au travers des ouvertures latérales 152 (figure 5). Tout en maintenant la tige de liaison 30 en place, par exemple à l'aide d'une pince, il visse ensuite la vis de verrouillage 160 dans le taraudage de l'ouverture d'introduction 155 du corps 150.

Le chirurgien serre alors la vis de verrouillage 160 de manière à ce que la tige de liaison 30 prenne appui sur la bague 140 afin d'immobiliser la liaison rotule.

En revanche, si le chirurgien souhaite désactiver la liaison rotule avant de mettre en place la tige de liaison 30, il utilise une clef de blocage 500 pour déplacer l'élément de blocage 130 depuis sa position de stockage jusqu'à sa position de blocage.

Comme le montrent les figures 7 à 9, une telle clef de blocage 500 présente préférentiellement un corps 501 cylindrique, une extrémité cylindrique 503 de plus petit diamètre à engager dans l'empreinte de réception 116 de la tête 112 de la partie d'ancrage 110, et une partie tubulaire 502 qui s'étend autour de l'extrémité cylindrique 503 de manière à pouvoir appuyer sur la face arrière de l'élément de blocage 130 sans interférer avec la bague 140.

Grâce à cette clef de blocage 500, le chirurgien peut dans un premier temps redresser la partie de montage 120 par rapport à la partie d'ancrage 110 (figure 7) en engageant l'extrémité cylindrique 503 de la clef de blocage 500 dans l'empreinte de réception 116 de la tête 112 de la partie d'ancrage 110. Les axes A1 et A2 se confondent ainsi avec l'axe principal.

Le chirurgien peut ensuite pousser l'élément de blocage 130 de manière que sa première rainure périphérique 132 se désengage de la nervure périphérique 154 du corps 150, et que sa seconde rainure périphérique 131 s'engage à son tour sur ladite nervure périphérique 154 (figures 8 et 10). Cette opération est rendue possible grâce aux fentes de l'élément de blocage 130 et de la bague 140 qui permettent à ces deux éléments de se déformer élastiquement radialement pour que l'élément de blocage 130 puisse passer de la position de stockage à la position de blocage. A ce stade, la liaison rotule est donc désactivée, de telle manière que les parties d'ancrage 110 et de montage 120 de l'implant 100 sont liées l'une à l'autre par une liaison pivot.

Le chirurgien procède ensuite de manière homologue pour fixer la tige de liaison 30, en engageant cette dernière dans le logement axial 151 du corps 150 et en vissant la vis de verrouillage 160 dans le taraudage de l'ouverture d'introduction 155 du corps 150, de manière à immobiliser la liaison pivot (figure 11).

Dans le second mode de réalisation de l'invention représenté sur les figures 12 à 15, la tête 212 de la partie d'ancrage 210 présente une forme d'hémisphère dont le sommet se raccorde au corps fileté 211 de la partie d'ancrage 210, et dont la base 213 est tournée vers l'arrière de la partie de montage 220. Cette base est plane, à l'exception d'une partie centrale creusée par ladite empreinte de réception 216.

La bague 240 présente en correspondance une forme sensiblement tubulaire, avec une face extérieure 244 sensiblement cylindrique de révolution autour de l'axe A2 et une face intérieure 242 en deux parties, dont une partie avant 248 d'accueil de la tête 212 de la partie d'ancrage 210 et une partie arrière 249 d'accueil de l'élément de blocage 230.

La partie avant 248 présente une forme de tronçon de sphère de diamètre égal, au jeu près, au diamètre de la tête 212 de la partie d'ancrage 210. Cette partie avant 248 constitue lesdits seconds moyens de liaison en ce sens qu'elle forme l'embout femelle de la liaison rotule.

La bague 240 est logée dans un logement qui est formé par l'ouverture 253 située au fond du logement axial 251 du corps 250 de la partie de montage 220.

Cette ouverture 253 présente plus précisément une partie arrière 259 cylindrique de révolution autour de l'axe A2, qui se prolonge sur une partie avant 358 tronconique dont le sommet est tourné vers la partie d'ancrage 210. Grâce à cette forme tronconique, lorsqu'une tige de liaison 30 appuie sur la face arrière 243 de la bague 240, cette dernière s'enfonce dans l'ouverture 253 et se comprime radialement sur la tête 210 de la partie d'ancrage, ce qui a pour effet d'immobiliser la liaison rotule.

Dans ce mode de réalisation de l'invention, les moyens de retenue de l'élément de blocage 230 en position de blocage et de stockage viennent de formation avec la bague 240. Ils sont en l'espèce formés par des rainures périphériques 245, 246 situées sur la partie arrière 249 de la face intérieure 242 de la bague 240. Ces deux rainures périphériques 245, 246 présentent des symétries de révolution autour de l'axe A2 et des sections triangulaires.

L'élément de blocage 230 présente quant à lui une forme de rondelle plate de diamètre intérieur supérieur à la plus grande dimension de l'empreinte de réception 216 de la tête 212 de la partie d'ancrage 210, et de diamètre extérieur égal, au jeu près, au diamètre des rainures périphériques 245, 246 de la bague 240.

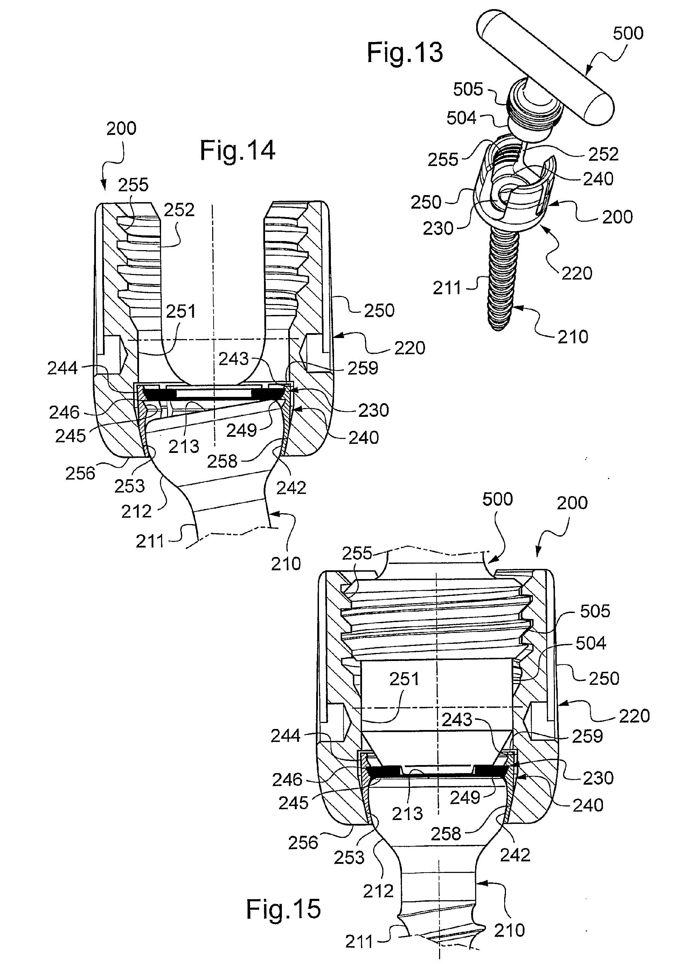

L'élément de blocage 230 est ainsi adapté à être bloqué, au choix, en position de stockage dans la rainure périphérique 246 arrière (figure 14) ou en position de blocage dans la rainure périphérique 245 avant (figure 15) de la bague 240, sans pour autant gêner l'insertion d'une clef allen dans l'empreinte de réception 216 de la tête 212 de la partie d'ancrage 210.

Le passage de l'élément de blocage 230 d'une position à l'autre est permis grâce à l'élasticité radiale de la bague 240, qui lui est conférée par quatre fentes qui s'étendent sur une partie de sa longueur et qui sont régulièrement réparties sur son pourtour.

Dans la position de stockage, la face avant de l'élément de blocage 230 est située à distance de la partie plane de la base 213 de la tête 212 de la partie d'ancrage 210, de manière à laisser la liaison rotule activée.

En revanche, en position de blocage, la face avant de l'élément de blocage 230 est située au contact de la base 213 de la tête 212 de la partie d'ancrage 210, de manière à désactiver la liaison rotule, de telle manière que les parties d'ancrage 210 et de montage 220 de l'implant 200 soient liées l'une à l'autre par une liaison pivot.

Ici, les rainures périphériques 245, 246 présentent des sections en forme de triangles rectangles dont les hypoténuses sont tournées vers l'arrière du corps 250 de la partie de montage 220, de manière que l'élément de blocage 230 peut être engagé à l'intérieur du logement axial 251 par l'arrière du corps 250 en position de stockage puis de blocage, mais qu'il ne peut ni passer de la position de blocage à la position de stockage, ni être extrait du corps 250. Il est ainsi parfaitement imperdable.

Comme le montrent les figures 13 et 15, le déplacement de l'élément de blocage 230 depuis sa position de stockage jusqu'à sa position de blocage est ici également réalisé au moyen d'une clef de blocage 500 adaptée à venir au contact de la face arrière de l'élément de blocage 230.

Telle que représentée sur la figure 15, cette clef de blocage 500 comporte un corps cylindrique 504 muni d'un filetage 505 identique au filetage de la vis de verrouillage 260. Il suffit donc de visser cette clef de blocage 500 dans le taraudage de l'ouverture d'introduction 255 du logement axial 251 pour déplacer l'élément de blocage 230 depuis sa position de stockage jusqu'à sa position de blocage. La force nécessaire pour déplacer l'élément de blocage 230 est ainsi réduite.

Dans le troisième mode de réalisation de l'implant rachidien 300 représenté sur les figures 16 à 18, l'élément de blocage 330 appartient à ladite partie d'ancrage 310.

Plus particulièrement, la tête 312 de la partie d'ancrage 310 comporte deux parties hémisphériques 313, 330 vissées l'une sur l'autre, dont une partie hémisphérique avant 113 raccordée par son sommet au corps fileté 311 de la partie d'ancrage 310, et une partie hémisphérique arrière qui constitue ledit élément de blocage 330.

La base de la partie hémisphérique avant 113 comporte à cet effet un plot fileté 317 dont la face d'extrémité présente en creux une empreinte de réception 316 d'une clef allen.

La base de l'élément de blocage 330 présente en correspondance un alésage taraudé 337 qui est vissé sur ce plot fileté 317. Le sommet de cet élément de blocage 330 présente en creux une empreinte de réception 336 d'une clef allen. Comme le montrent les figures 17 et 18, l'alésage taraudé 337 et l'empreinte de réception 336 communiquent ici l'un avec l'autre.

Dans ce mode de réalisation de l'invention, les moyens de retenue de l'élément de blocage 330 viennent de formation avec la partie d'ancrage 310 et sont formés par le plot fileté 317. Ils sont adaptés à fixer l'élément de blocage 330 en position de stockage lorsque ce dernier est vissé jusqu'en butée sur le plot fileté 317, ou en position de blocage lorsque ce dernier est en partie dévissé de ce plot fileté 317.

En correspondance, l'ouverture 353 située au fond du logement axial 351 du corps 350 présente une partie arrière 359 cylindrique de révolution autour de l'axe A2, qui se prolonge sur une partie avant 358 en forme de tronçon de sphère de diamètre égal, au jeu près, au diamètre de la partie hémisphérique avant 313 de la tête 312 de la partie d'ancrage 310.

La partie avant 358 de l'ouverture 353 forme ainsi une partie desdits seconds moyens de liaison en ce sens qu'elle constitue une partie de l'embout femelle de la liaison rotule.

La partie arrière 359 de l'ouverture 353 permet quant à elle de désactiver cette liaison rotule lorsque l'élément de blocage 330 est en partie dévissé du plot fileté 317 (figure 18), de telle manière que les parties d'ancrage 310 et de montage 320 de l'implant 300 soient liées l'une à l'autre par une liaison pivot.

En effet, dans cette position, les contours circulaires des bases des deux parties hémisphériques 313, 330 de la tête 312 de la partie d'ancrage 310 s'étendent dans la partie arrière 359 cylindrique de l'ouverture 353, ce qui bloque la liaison rotule en liaison pivot.

Telle que représentée sur la figure 16, la bague 340 présente ici une forme sensiblement tubulaire, avec une face extérieure 344 cylindrique de révolution autour de l'axe A2 de diamètre égal, au jeu près, au diamètre du logement axial 351. Elle présente une face intérieure en deux parties, dont une partie arrière 341 cylindrique de révolution autour de cet axe A2, et une partie avant 342 tronconique engagée sur l'élément de blocage 330.

La mise en place de l'implant rachidien 300 sur une vertèbre 10, 20 est réalisée par le chirurgien de la manière suivante.

Après avoir extrait la vis de verrouillage 360 du corps 350 de la partie de montage 320, le chirurgien qui a accès à l'empreinte de réception 316 de la tête 312 de la partie d'ancrage 310 de l'implant 300 visse cette partie d'ancrage 310 dans la vertèbre 10, 20 du patient.

Il estime ensuite s'il faut ou non désactiver la liaison rotule de l'implant rachidien 300.

S'il souhaite laisser la liaison rotule active (figure 17), il s'assure que l'élément de blocage 330 est vissé jusqu'en butée sur le plot fileté 317, en position de stockage. Il place ensuite la tige de liaison 30 préalablement cintrée dans le logement axial 351 du corps 350, au travers des ouvertures latérales 352. Puis, il visse la vis de verrouillage 360 dans le taraudage de l'ouverture d'introduction 355 du corps 350, et serre cette vis de verrouillage 360 de manière à ce que la tige de liaison 30 prenne appui sur la bague 340 et immobilise ainsi la liaison rotule.

En revanche, si le chirurgien souhaite désactiver la liaison rotule, il utilise une simple clef allen pour déplacer l'élément de blocage 330 depuis sa position de stockage jusqu'à sa position de blocage.

Le chirurgien dévisse à cet effet l'élément de blocage 330 sur une partie de la longueur du plot fileté 317 (figure 18), de telle manière que l'élément de blocage 330 s'étende en majeure partie dans le logement axial 351, en avoisinant les fonds des ouvertures latérales 352 du corps 350. De cette manière, la tête 312 de la partie d'ancrage 310 est bloquée en liaison pivot par rapport à la partie de montage 320.

Le chirurgien procède ensuite de manière identique pour fixer la tige de liaison 30, en engageant cette dernière dans le logement axial 351 et en vissant la vis de verrouillage 360 dans le taraudage de l'ouverture d'introduction 355 du corps 350, de manière à immobiliser la liaison pivot (figure 11).

Dans le quatrième mode de réalisation de l'invention représenté sur les figures 19 à 22, la tête 612 de la partie d'ancrage 610 présente deux parties hémisphériques de diamètres différents pour délimiter entre elles un épaulement annulaire 615 autour de l'axe A1. Ces deux parties hémisphériques sont ici rainurées de manière à accroître leur adhérence.

En correspondance, l'ouverture 653 située au fond du logement axial 651 du corps 650 de la partie de montage 620 présente une forme de tronçon de sphère pour former l'embout femelle de la liaison rotule.

Telle que représentée sur la figure 21, la bague 640 présente une forme sensiblement tubulaire. Elle est bordée, du côté de son extrémité avant, par une couronne extérieure. Elle présente intérieurement, du côté de son extrémité avant, une partie 642 sphérique engagée sur la partie hémisphérique arrière de la tête 612 de la partie d'ancrage 610.

L'élément de blocage 630 présente quant à lui une forme d'anneau. Il présente intérieurement, du côté de son extrémité arrière, une rainure 635 de diamètre intérieur égal, au jeu près, au diamètre extérieur de la bague 640. Cet élément de blocage 630 peu ainsi être emmanché sur la bague 640 de telle manière qu'il est adapté à coulisser le long de la face extérieure de la bague 640 jusqu'à une position de butée dans laquelle sa rainure 635 vient au contact de la couronne extérieure de la bague 640.

Dans ce mode de réalisation de l'invention, les moyens de retenue 654 de l'élément de blocage 630 viennent de formation avec le corps 650 de la partie de montage 620. Ils comportent en l'espèce un taraudage qui s'étend dans le logement axial 651.

L'élément de blocage 630 présente en correspondance, sur sa face extérieure, un filetage 631 à visser dans le taraudage 654 du corps 650.

Tel que représenté sur les figures, les moyens de retenue 654 de l'élément de blocage 630 comportent en outre une gorge périphérique, située à l'arrière du taraudage, qui accueille un circlip 670 de maintien de l'élément de blocage 630 dans le logement axial 651 du corps 650. Ce circlip est en l'espèce formé par un simple anneau fendu.

Ce circlip 670 permet ainsi de bloquer l'élément de blocage 630 en position de stockage (figure 21) lorsque le filetage 631 de l'élément de blocage 630 est dévissé du taraudage du corps 650. Dans cette position de stockage, la face avant de l'élément de blocage 630 est située à distance de l'épaulement annulaire 615 de la tête 612 de la partie d'ancrage 610, de manière à laisser la liaison rotule activée.

En revanche, en position de blocage, lorsque l'élément de blocage 630 est vissé dans le taraudage 654 du corps 650, la face avant de l'élément de blocage 630 est située au contact de l'épaulement annulaire 615 de la tête 612 de la partie d'ancrage 610, de manière à désactiver la liaison rotule. Dans cette position, le corps 650 est maintenu en position fixe par rapport à la partie d'ancrage 610.

La mise en place de l'implant rachidien 600 sur une vertèbre 10, 20 est réalisée par le chirurgien en utilisant un outil de blocage 700 du type de celui représenté sur la figure 20.

Un tel outil de blocage 700 comporte ici une tige intérieure 710 montée mobile en rotation dans un tube extérieur 720.

Le tube extérieur 720 comporte, du côté de son extrémité avant, deux ailes latérales 721 de formes identiques, en négatif, à celles des ouvertures latérales 652 du corps 650. Ce tube extérieur 720 permet ainsi, lors de l'installation de l'implant rachidien 600 sur une vertèbre 10, 20, de maintenir le corps 650 de l'implant en position fixe par rapport aux vertèbres.

La tige intérieure 710 comporte quant à elle quatre ergots 711 en forme de créneaux qui s'élèvent à partir de son extrémité avant pour s'engager dans des échancrures prévus en correspondance sur l'extrémité arrière de l'élément de blocage 630. Cette tige intérieure 710 permet ainsi, lors de l'installation de l'implant rachidien 600 sur une vertèbre 10, 20, de visser l'élément de blocage 630 dans l'alésage du corps 650 de l'implant, de manière à placer l'élément de blocage 630 en position de blocage.

Cette tige intérieure 710 est en outre équipée d'un téton avant 712 permettant de redresser la partie de montage 620 dans l'axe de la partie d'ancrage 610 de l'implant rachidien 600 avant que l'élément de blocage 630 n'immobilise la liaison rotule.

## Revendications

1. Implant rachidien (100) comprenant :
- une partie d'ancrage (110) à une vertèbre (10, 20), comportant un corps fileté (111) s'étendant suivant un premier axe (A1) et des premiers moyens de liaison (112), et
- une partie de montage (120) comprenant, d'une part, intérieurement un logement axial (151) s'étendant suivant un deuxième axe (A2) et débouchant vers l'extérieur par deux ouvertures latérales (152) en regard et en forme de berceaux pour accueillir transversalement une tige de liaison (30), ce logement axial (151) étant délimité du côté de la partie d'ancrage (110) par un fond (156), et, d'autre part, des seconds moyens de liaison (140, 153) qui coopèrent avec lesdits premiers moyens de liaison (112) pour former une liaison rotule entre ladite partie d'ancrage (110) et ladite partie de montage (120),
- des moyens de retenue (154) qui sont situés dans un espace compris entre le fond des berceaux formés par lesdites deux ouvertures latérales (152) et le fond (156) du logement axial (151), et qui sont adaptés à fixer un élément de blocage (130) dans une position fixe dite de blocage dans laquelle la liaison rotule formée par lesdits premiers et seconds moyens de liaison (112, 140, 153) est bloquée pour rendre lesdites parties d'ancrage (110) et de montage (120) fixes en rotation l'une par rapport à l'autre autour d'au moins deux axes orthogonaux (A3, A4).
**caractérisé en ce que** lesdits moyens de retenue (154) sont agencés de telle manière qu'ils sont capables de fixer ledit élément de blocage (130) en position de blocage seulement lorsque ledit deuxième axe (A2) est confondu avec ledit premier axe (A1) et **en ce que** ledit élément de blocage (130) est distinct des seconds moyens de liaison (140, 153).

2. Implant rachidien (100) selon la revendication précédente, dans lequel lesdits moyens de retenue (154) sont adaptés à stocker ledit élément de blocage (130) dans une position fixe dite de stockage, distincte de la position de blocage, dans laquelle la liaison rotule formée par lesdits premiers et seconds moyens de liaison (112, 140, 153) est laissée libre.

3. Implant rachidien (100) selon l'une des revendications précédentes, dans lequel, lorsque la liaison rotule est bloquée, lesdites parties d'ancrage (110) et de montage (120) sont fixes l'une par rapport à l'autre.

4. Implant rachidien (100) selon l'une des revendications 1 et 2, dans lequel, lorsque la liaison rotule est bloquée, lesdites parties d'ancrage (110) et de montage (120) sont libres de pivoter autour d'un seul axe principal (A1), orthogonal auxdits deux axes orthogonaux (A3, A4).

5. Implant rachidien (100) selon l'une des revendications précédentes, dans lequel lesdits premiers moyens de liaison comportent une tête (112) au moins en partie sphérique et dans lequel lesdits seconds moyens de liaison comportent une bague (140) dont une face intérieure (142) est en appui contre ladite tête (112).

6. Implant rachidien (100) selon la revendication précédente, dans lequel ledit logement axial (151) débouche vers l'extérieur par deux ouvertures latérales (152) en regard et en forme de berceaux, et dans lequel ladite bague (140) présente une face d'extrémité (143) qui s'étend au-dessus des fonds des berceaux formés par lesdites deux ouvertures latérales (152).

7. Implant rachidien (100) selon l'une des revendications précédentes, dans lequel ledit logement axial (151) débouche vers l'extérieur à l'opposé du fond (156) par une ouverture d'introduction (155) et ladite partie de montage (120) comporte, à proximité de cette ouverture d'introduction (155), un filetage (154) qui coopère avec un filetage (162) complémentaire d'une vis de verrouillage (160) pour immobiliser ladite tige de liaison (30) et ladite liaison rotule.

8. Implant rachidien (100) selon l'une des revendications précédentes, comportant un élément de blocage (130) adapté à coopérer avec lesdits moyens de retenue (154) pour être fixé dans ladite position de blocage.

9. Implant rachidien (300) selon la revendication précédente, dans lequel ledit élément de blocage (330) appartient à ladite partie d'ancrage (310).

10. Implant rachidien (300) selon les revendications 5 et 9, dans lequel la tête (312) de la partie d'ancrage (310) comporte deux parties (313, 330) vissées l'une sur l'autre, dont une première partie constitue ledit élément de blocage (330).

11. Implant rachidien (300) selon la revendication précédente, dans lequel le fond (356) qui délimite le logement axial (351) du côté de la partie d'ancrage (310) présente une ouverture (353) par laquelle ledit logement axial (351) débouche vers l'extérieur, cette ouverture (353) présentant une partie cylindrique qui se prolonge sur une partie sphérique ou conique, et dans lequel ledit élément de blocage (330), lorsqu'il est en position de blocage, est situé au contact de ladite partie cylindrique de l'ouverture (353).

12. Implant rachidien (100 ; 200) selon la revendication 8, dans lequel ledit élément de blocage (130 ; 230) appartient à ladite partie de montage (120 ; 220).

13. Implant rachidien (100) selon la revendication précédente, dans lequel ledit élément de blocage (130) est constitué par un anneau.

14. Implant rachidien (100) selon l'une des deux revendications précédentes, dans lequel ledit élément de blocage (130) et ladite partie d'ancrage (110) comportent des faces de butée (115, 135) qui, lorsque ledit élément de blocage (130) est en position de blocage, sont situées au contact ou à proximité immédiate l'une de l'autre.

## Patentansprüche

1. Wirbelimplantat (100), das umfasst:
- einen Verankerungsabschnitt (110) in einer Wirbel (10,20), der ein Gewindeteil (111) umfasst, das sich entlang einer ersten Achse (A1) erstreckt, und erste Verbindungsmittel (112), und
- einen Montageabschnitt (120), der zum einen in seinem Innenraum eine axiale Aufnahme (151) umfasst, die sich entlang einer zweiten Achse (A2) erstreckt und über zwei seitliche, gegenüberliegende und wiegenförmige Öffnungen (152) nach außen mündet, um einen Verbindungsstift (30) quer aufzunehmen, wobei diese axiale Aufnahme (151) auf der Seite des Verankerungsabschnitts (110) durch einen Boden (156) abgegrenzt ist, und zum anderen durch zweite Verbindungsmittel (140,153), die mit den ersten Verbindungsmitteln (112) zusammenwirken, um eine Gelenkverbindung zwischen dem Verankerungsabschnitt (110) und dem Montageabschnitt (120) zu bilden,
- Rückhaltemittel (154), die sich in einem Raum zwischen dem Boden der durch die zwei seitlichen Öffnungen (152) gebildeten Wiegen und dem Boden (156) der axialen Aufnahme (151) befinden, und sich dazu eignen, ein Klemmelement (130) in einer festen, als Klemmposition bezeichneten Position zu fixieren, in der die Gelenkverbindung aus den ersten und zweiten Verbindungsmitteln (112, 140, 153) blockiert ist, um die Verankerungsabschnitte (110) und Montageabschnitte (120) um mindestens zwei orthogonale Achsen (A3, A4) zueinander drehfest zu machen, **dadurch gekennzeichnet, dass** die Rückhaltemittel (154) derart angeordnet sind, dass sie das Klemmelement (130) nur dann in der Klemmposition fixieren können, wenn die zweite Achse (A2) mit der ersten Achse (A1) übereinstimmt und dadurch, dass sich das Klemmelement (130) von den zweiten Verbindungsmitteln (140, 153) unterscheidet.

2. Wirbelimplantat (100) nach dem vorausgehenden Anspruch, bei dem sich die Rückhaltemittel (154) dazu eignen, das Klemmelement (130) in einer festen, sogenannten Lagerposition zu lagern, die sich von der Klemmposition unterscheidet, wobei in der Lagerposition die aus den ersten und zweiten Verbindungsmitteln (112, 140, 153) bestehende Gelenkverbindung frei beweglich bleibt.

3. Wirbelimplantat (100) nach einem der vorausgehenden Ansprüche, bei dem, wenn die Gelenkverbindung blockiert ist, die Verankerungs- (110) und Montageabschnitte (120) zueinander unbeweglich sind.

4. Wirbelimplantat (100) nach einem der Ansprüche 1 und 2, bei dem, wenn die Gelenkverbindung blockiert ist, die Verankerungs- (110) und Montageabschnitte (120) um eine einzige Hauptachse (A1), die zu den zwei orthogonalen Achsen (A3, A4) orthogonal ist, schwenkbar sind.

5. Wirbelimplantat (100) nach einem der vorausgehenden Ansprüche, bei dem die ersten Verbindungsmittel einen zumindest teilweise sphärischen Kopf (112) umfassen und bei dem die zweiten Verbindungsmittel einen Ring (140) umfassen, dessen eine Innenfläche (142) an diesem Kopf (112) aufliegt.

6. Wirbelimplantat (100) nach vorausgehendem Anspruch, bei dem die axiale Aufnahme (151) über zwei seitliche, gegenüberliegende und wiegenförmige Öffnungen (152) nach außen mündet, und bei dem dieser Ring (140) eine Endfläche (143) aufweist, die sich über den Böden der aus den zwei seitlichen Öffnungen (152) gebildeten Wiegen erstreckt.

7. Wirbelimplantat (100) nach einem der vorausgehenden Ansprüche, bei dem die axiale Aufnahme (151) über eine Einführöffnung (155) dem Boden (156) gegenüberliegend nach außen mündet, und der Montageabschnitt (120) in der Nähe dieser Einführöffnung (155) ein Gewinde (154) umfasst, das mit einem komplementären Gewinde (162) einer Spannschraube (160) zusammenwirkt, um den Verbindungsstift (30) und die Gelenkverbindung zusammen zu blockieren.

8. Wirbelimplantat (100) nach einem der vorausgehenden Ansprüche, das ein Klemmelement (130) umfasst, das mit den Rückhaltemitteln (154) zusammenwirken kann, um in der Klemmposition fixiert zu werden.

9. Wirbelimplantat (300) nach vorausgehendem Anspruch, bei dem das Klemmelement (330) ein Teil des Verankerungsabschnitts (310) ist.

10. Wirbelimplantat (300) nach den Ansprüchen 5 und 9, bei dem der Kopf (312) des Verankerungsabschnitts (310) zwei aufeinander geschraubte Abschnitte (313,330) umfasst, wobei ein erster dieser Abschnitte das Klemmelement (330) bildet.

11. Wirbelimplantat (300) nach vorausgehendem Anspruch, bei dem der Boden (356), der die axiale Aufnahme (351) auf der Seite des Verankerungsabschnitts (310) abgrenzt, eine Öffnung (353) aufweist, durch die die axiale Aufnahme (351) nach außen mündet, wobei diese Öffnung (353) einen zylinderförmigen Abschnitt aufweist, der in einen sphärischen oder konischen Abschnitt übergeht, und bei dem das Klemmelement (330), wenn es in seiner Klemmposition ist, den zylinderförmigen Abschnitt der Öffnung (353) berührt.

12. Wirbelimplantat (100; 200) nach Anspruch 8, bei dem das Klemmelement (130; 230) Teil des Montageabschnitts (120; 220) ist.

13. Wirbelimplantat (100) nach vorausgehendem Anspruch, bei dem das Klemmelement (130) aus einem Ring besteht.

14. Wirbelimplantat (100) nach einem der zwei vorausgehenden Ansprüche, bei dem das Klemmelement (130) und der Verankerungsabschnitt (110) Anschlagflächen (115, 135) aufweisen, die sich, wenn das Klemmelement (130) in der Klemmposition ist, berühren bzw. in unmittelbarer Nähe zueinander befinden.

## Claims

1. A spinal implant (100) comprising:
• an anchoring part (110) adapted to be anchored to a vertebra (10, 20), comprising a threaded body (111) extending along a first axis (A1) and first connecting means (112); and
• a mounting part (120) comprising both an internal axial housing (151) extending along a second axis (A2) and open to the outside via two facing lateral openings (152) in the form of cradles for receiving transversely a connecting rod (30), this axial housing (151) being terminated beside the anchoring part (110) by a bottom (156), and also second connecting means (140, 153) that cooperate with said first connecting means (112) to form a ball-joint connection between said anchoring part (110) and said mounting part (120);
• retaining means (154) that are situated in a space between the bottom of the cradles formed by the two lateral openings (152) and the bottom (156) of the axial housing (151) and that are adapted to fasten a locking member (130) in a stationary so-called locking position in which the ball-joint connection formed by said first and second connecting means (112, 140, 153) is locked to fasten said anchoring part (110) and said mounting part (120) in rotation relative to each other about at least two orthogonal axes (A3, A4), the implant being **characterized in that** said retaining means (154) are arranged in such a manner as to be capable of fastening said locking member (130) in the locking position only when said second axis (A2) coincides with said first axis (A1) and **in that** said locking member (130) is separate from said second connecting means (140, 153).

2. A spinal implant (100) according to the preceding claim, wherein said retaining means (154) are adapted to store said locking member (130) in a stationary "storage" position that is separate from the locking position and in'which the ball-joint connection formed by said first and second connecting means (112, 140, 153) is left free.

3. A spinal implant (100) according to either preceding claim, wherein, when the ball-joint connection is locked, said anchoring part (110) and said mounting part (120) are stationary relative to each other.

4. A spinal implant (100) according to claim 1 or claim 2, wherein, when the ball-joint connection is locked, said anchoring part (110) and said mounting part (120) are free to pivot about a single main axis (A1) orthogonal to said two orthogonal axes (A3, A4).

5. A spinal implant (100) according to any preceding claim, wherein said first connecting means include a head (112) that is at least partly spherical and wherein said second connecting means include a ring (140) with an inside face (142) that bears against said head (112).

6. A spinal implant (100) according to the preceding claim, wherein said axial housing (151) is open to the outside via two facing lateral openings (152) in the form of cradles and wherein said ring (140) has an end face (143) that lies above the bottoms of the cradles formed by said two lateral openings (152).

7. A spinal implant (100) according to any preceding claim, wherein said axial housing (151) is open to the outside at its end opposite from the bottom (156) via an introduction opening (155) and said mounting part (120) includes near this introduction opening (155) a thread (154) that cooperates with a complementary thread (162) of a locking screw (160) to prevent said connecting rod (30) and said ball-joint connection from moving.

8. A spinal implant (100) according to any preceding claim, including a locking member (130) adapted to cooperate with said retaining means (154) to be fastened in said locking position.

9. A spinal implant (300) according to the preceding claim, wherein said locking member (330) is part of said anchoring part (310).

10. A spinal implant (300) according to claims 5 and 9, wherein the head (312) of the anchoring part (310) includes two parts (313, 330) screwed together, a first of which parts constitutes said locking member (330).

11. A spinal implant (300) according to the preceding claim, wherein the bottom (356) that terminates the axial housing (351) beside the anchoring part (310) has an opening (353) through which said axial housing (351) is open to the outside, this opening (353) having a cylindrical part that lies over a spherical or conical part, and wherein said locking member (330), when it is in the locking position, is situated in contact with said cylindrical part of the opening (353).

12. A spinal implant (100; 200) according to claim 8, wherein said locking member (130; 230) is part of said mounting part (120; 220).

13. A spinal implant (100) according to the preceding claim, wherein said locking member (130) is an annulus.

14. A spinal implant (100) according to either of the preceding two claims, wherein said locking member (130) and said anchoring part (110) include abutment faces (115; 135) which, when said locking member (130) is in the locking position, are situated in contact with or in the immediate vicinity of each other.
